(19) Europäisches Patentamt
European Patent Office
Office européen des brevets

(11) Numéro de publication: **0 252 804**
**B1**

(12) **FASCICULE DE BREVET EUROPEEN**

(45) Date de publication du fascicule du brevet: **07.11.90**

(51) Int. Cl.$^5$: **C 08 F 10/00,** C 08 F 4/64

(21) Numéro de dépôt: **87401508.4**

(22) Date de dépôt: **30.06.87**

(54) **Procédé de traitement de catalyseurs sphériques de polymérisation des oléfines, application du catalyseur obtenu à la polymérisation des oléfines.**

(30) Priorité: **08.07.86 FR 8609930**

(43) Date de publication de la demande:
**13.01.88 Bulletin 88/02**

(45) Mention de la délivrance du brevet:
**07.11.90 Bulletin 90/45**

(84) Etats contractants désignés:
**AT BE CH DE ES FR GB IT LI LU NL SE**

(56) Documents cités:
**EP-A- 232 643**
**FR-A-2 474 039**
**GB-A-2 068 981**
**US-A-4 312 784**

**Le dossier contient des informations techniques présentées postérieurement au dépôt de la demande et ne figurant pas dans le présent fascicule.**

(73) Titulaire: **ATOCHEM**
**4 & 8, Cours Michelet La Défense 10**
**F-92800 Puteaux (FR)**

(72) Inventeur: **Brun, Claude**
**Clos St Pierre**
**F-64320 Idron Bizanos (FR)**
Inventeur: **Cheux, Auguste**
**18, Route d'Artix**
**F-64370 Arthez-de-Bearn (FR)**
Inventeur: **Avaro, Michel**
**Domaine de St Léon 15, Rue de la Grange**
**F-64000 Pau (FR)**

(74) Mandataire: **Foiret, Claude et al**
**ATOCHEM Département Propriété Industrielle**
**F-92091 Paris la Défense 10 Cédex 42 (FR)**

**EP 0 252 804 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne un procédé de traitement de catalyseur sphérique de polymérisation des oléfines permettant de lui conserver sa morphologie pendant la polymérisation. Le procédé consiste à prépolymériser de l'éthylène, étant entendu que sous la dénomination éthylène on entend également les mélanges d'éthylène et d'oléfines, jusqu'à un degré d'avancement réduit, un produit, appelé sphéroprotecteur, à effet conservateur de sphéricité constitué essentiellement d'un alkylaluminium halogéné spéfique, étant associé aux composants au plus tard après le prépolymérisation. Un tel catalyseur modifié est particulièrement adapté pour la fabrication de polyéthylène linéaire en poudre à morphologie sphérique.

Afin de fabriquer des polyéthylènes linéaires sphériques: homopolymères d'éthylène et copolymères éthylène-alpha oléfine, il est connu d'utiliser des catalyseurs sphériques à base de métal de transition et plus particulièrement de titane. Toutefois, dans les conditions des procédés industriels de polymérisation, la morphologie sphérique du catalyseur est rapidement détruite. Les particules sphériques du catalyseur se détruisent rapidement en particules à morphologie mal définie, du type par exemple granulaire, ce qui conduit à des polymères plus ou moins granulaires de faible coulabilité, caractéristique liée à la morphologie plus ou moins sphérique du polymère obtenu. Ces polymères sont également riches en très fines particules, inférieures à 100 µm (microns), conduisant à des problèmes de sécurité et entraînant des difficultés dans le procédé de fabrication.

Dans le EP—A—232643 est proposé un traitement de catalyseur sphérique de polymérisation des oléfines permettant de lui conserver sa morphologie pendant la polymérisation. Le procédé consiste à prépolymériser de l'éthylène, un sphéroprotecteur à effet de conservateur de sphéricité résultant de la réaction d'un alkylaluminium et d'un donner d'électrons étant associé aux composants au plus tard après la prépolymérisation. Cette technique, bien qu'efficace, présente l'inconvénient d'avoir à utiliser un donneur d'électrons, produit connu en catalyse Ziegler, pour nuire à l'activité des catalyseurs et qui, pour cette raison, demande à être employé avec précaution.

Le procédé objet de l'invention, selon lequel la morphologie sphérique du catalyseur est conservée pendant la polymérisation de la ou des oléfines, confirmant le principe selon lequel les polymères formés sont la réplique morphologique du catalyseur, conduit à un prépolymère catalyseur permettant d'obtenir par polymérisation en suspension ou en phase gazeuse d'éthylène ou de mélange d'éthylène et d'alpha-oléfine un polyéthylène linéaire en poudre à morphologie sphérique. Cette morphologie sphérique est encore déterminée subjectivement. Sans en faire une définition exclusive, on peut admettre qu'une poudre de polyéthylène possède une morphologie sphérique quand les particules de poudre sont en moyenne, par examen microscopique optique, sensiblement sphériques, symétriques, sans élongation, à surface lisse au grossissement 20. Ce mode d'appréciation semble être confirmé par les tentatives d'analyses expérimentales de J. K. Beddow, Fine Particle Research Group., Chemical and Materials Engineering, University of Iowa dans "Proceedings of the ACS Division of Polymeric Materials: Science and Engineering" Volume 53 Fall Meeting 1985—Chicago pages 261—262.

Le procédé de traitement du catalyseur sphérique de polymérisation des oléfines est caractérisé en ce qu'on effectue, en sa présence, une prépolymérisation en présence d'un cocatalyseur choisi parmi les alkylaluminiums, au moins partiellement en suspension, de l'éthylène jusqu'à un degré de prépolymérisation adapté au procédé de polymérisation dans lequel le prépolymère sea mis ultérieurement en oeuvre, un sphéroprotecteur sous forme d'alkylaluminium halogéne étant associé aux composants au plus tard après la prépolymérisation.

Par addition de tous les éléments entrant dans la composition du sphéroprotecteur, on admet qu'il répond à la formule

$$Al\ R'_mR''_nCl_pH_q$$

dans laquelle

$0{,}05<p\leq 1$
$0<m<2{,}95$
$0<n<2{,}95$
$0\leq q\leq 1$

sachant que $m+n+p+q=3$ et R' et R'', identiques ou différents, sont des radicaux hydrocarbonés linéaires ramifiés ou cycliques contenant de 1 à 14 atomes de carbone.

Ce sphéroprotecteur tel que défini peut être un alkylaluminium monohalogéné, mais préférentiellement il résulte du mélange de deux alkylaluminiums, l'un étant un alkylaluminium non halogéné, l'autre un alkylaluminium mono halogéne, étant entendu qu'il peut s'agir du mélange d'un ou plusieurs alkylaluminiums non halogénés et d'un ou plusieurs alkylaluminiums monohalogénés. Les radicaux alkyls des deux composés de l'aluminium sont indifféremment choisis parmi ceux définis précédemment. Il est fabriqué par mélange des deux composés, dans les conditions connues et adaptées à la manipulation délicate de ces produits, en proportions telles que soit respectée la définition de la formule admise. On obtient un mélange liquide.

Les alkylaluminiums entrant dans la fabrication du sphéroprotecteur sont connus en eux mêmes et ils sont choisis parmi ceux utilisés habituellement comme cocatalyseur. Ils répondent aux formules générales $AlR_x H_z$ et $Al R_xCl_yH_z$ ou x+z et x+y+z=3 et dans lesquelles R représente un radical hydrocarboné linéaire, ramifié ou cyclique contenant de 1 à 14 atomes de carbone. A titre d'exemples on peut citer:

$$Al(C_4H_9)_3, Al(C_4H_9)_2H, Al(C_2H_5)_3, Al(C_6H_{13})_3, Al(C_8H_{17})_3,$$

$$Al(C_2H_5)_2 \text{ et } Al(C_2H_5)_2Cl, Al(C_6H_{13})_2Cl, AlCl(C_4H_9iso)_2.$$

Le prépolymère obtenu selon le procédé est utilisé comme catalyseur dans les procédés de polymérisation en suspension et en phase gazeuse, soit en lit agité soit en lit fluidisé. Selon le procédé de polymérisation envisagé, la prépolymérisation en présence du catalyseur doit être plus ou moins poussée. Dans le cas où le prépolymère sera utilisé comme catalyseur dans un procédé en suspension, le degré de prépolymérisation est de préférence inférieure à 100, par contre pour un procédé en phase gazeuse, le degré de prépolymérisation est de préférence supérieure à 50 et telle que le prépolymère formé représente au plus 10% en poids du polymère final.

Le catalyseur sphérique initial de polymérisation des oléfines est un produit connu en lui-même. Il est habituellement le résultat de la combinaison d'au moins un composé de métal de transition, un composé du magnésium, un halogène et éventuellement un donneur ou accepteur d'électrons et de tous autre composé utilisable dans ces types de catalyseur.

Le composé de métal de transition est généralement choisi parmi les composés de formule $Me(OR)_nX_{m-n}$ dans laquelle:

Me est le vanadium, le chrome et plus particulièrement le titane;

X est le brome, l'iode et plus particulièrement le chlore;

R est un radical hydrocarboné aliphatique ou aromatique de $C_1$ à $C_{14}$, ou COR' avec R' un radical hydrocarboné aliphatique ou aromatique de $C_1$ à $C_{14}$.

"m" correspond à la valence du métal de transition et "n" est une valeur inférieure ou égale à "m".

Le composé de métal de transition particulièrement recommandé est choisi parmi les composés du titane de formule $Ti(OR)_x\ Cl_{4-x}$, R étant défini ci-dessus, x étant compris entre 0 et 4.

Le composé du magnésium est habituellement choisi parmi les composés de formule $Mg(OR)_nX_{2-n}$ dans laquelle X est le brome, l'iode et plus particulièrement le chlore, R est l'hydrogène ou un radical alkyl ou cycloalkyl et "n" est inférieure ou égale à 2.

Le donneur ou accepteur d'électrons est un composé organique liquide ou solide connu pour entrer dans la composition de ces catalyseurs. Le donneur d'électrons peut être un composé mono ou polyfonctionnel avantageusement choisi parmi les acides carboxyliques aliphatiques ou aromatiques et leurs esters alcoyliques, les éthers aliphatiques ou cycliques, les cétones, les esters vinyliques, les dérivés acryliques, en particulier acrylates ou méthacrylates d'alcoyle, et les silanes. Conviennent notamment comme donneurs d'électrons les composés tels que paratoluate de méthyle, benzoate d'éthyle, acétate d'éthyle ou de butyle, éther éthylique, para-anisate d'éthyle, dibutylphtalate, dioctylphtalate, diisobutylphtalate, tétrahydrofuranne, dioxane, acétone, méthylisobutylcétone, acétate de vinyle, méthacrylate de méthyle, et les silanes tels que phényltriéthoxysilane, les alcoxysilanes aromatiques ou aliphatiques.

L'accepteur d'électrons est un acide de Lewis, choisi de préférence parmi les chlorures d'aluminium, le trifluorure de bore, le chloranile ou encore les alcoylaluminiums et alcoylmagnésiums.

Dans un mode préféré de prépolymérisation en suspension, sous agitation en régime turbulent, on prépolymérise l'éthylène, en présence éventuellement d'un limitateur de chaîne et/ou d'un cocatalyseur choisi parmi les alkylaluminiums connus pour cet emploi, à une température comprise entre 0 et 110°C, de préférence entre 20 et 60°C, pour une pression totale inférieure à 20 bars absolus constituée essentiellement de gaz inerte tel que l'azote. Afin de conserver au maximum la morphologie sphérique initiale du catalyseur, il est recommandé de controler l'alimentation en monomère dans le réacteur. Une vitesse moyenne d'alimentation favorable est inférieure ou égale à 500 $Nl.\times h^{-1}\times g^{-1}$ de catalyseur. La prépolymérisation en suspension est poursuivie jusqu'à un degré de prépolymérisation adapté au procédé de polymérisation ultérieur, le degré de prépolymérisation étant défini par le rapport de la somme du poids de prépolymère formé plus le poids de catalyseur mis en oeuvre sur le poids de catalyseur mis en oeuvre.

A un stade quelconque de la prépolymérisation on ajoute aux composants le sphéroprotecteur. Le sphéroprotecteur peut être introduit dans le milieu réactionnel de prépolymérisation. Il peut également être avantageusement additionné au prépolymère après la prépolymérisation soit directement dans le milieu réactionnel, soit au prépolymère stocké en suspension ou à l'état sec sous gaz inerte.

Dans un autre mode préféré de prépolymérisation en suspension sous agitation en régime turbulent, on procède à la prépolymérisation dans les conditions décrites précédemment jusqu'à un degré d'avancement réduit de prépolymérisation, de préférence inférieure à 20 g de polymère par gramme de catalyseur.

A ce stade, le prépolymère est isolé puis repris dans un système de prépolymérisation en phase gazeuse de façon à passer du degré d'avancement réduit de polymérisation au degré de prépolymérisation adapté au procédé de polymérisation ultérieur.

Cette partie de prépolymérisation en phase gazeuse s'effectue dans les conditions habituelles au procédé de polymérisation de l'éthylène en phase gazeuse. On peut, par exemple, associer dans un réacteur le prépolymère à degré d'avancement réduit à une charge de polyoléfine de granulomètrie moyenne inférieure ou égale à 3000 et de préférence inférieure ou égale à 1000 μm (microns), en présence de préférence d'un cocatalyseur tel que défini précédemment. Après homogénéisation, on poursuit la prépolymèrisation par introduction de monomère, de préférence l'éthylène, ou un mélange d'éthylène et de butène. De façon préférentielle la prépolymérisation en phase gazeuse est réalisée à une température comprise entre 30 et 110°C sous une pression totale inférieure ou égale à 20 bars.

Cette prépolymérisation en phase gazeuse est poursuivie jusqu'à l'obtention d'un degré de prépolymérisation adapté au procédé de polymérisation ultérieur. Afin de conserver au maximum la morphologie sphérique initiale du catalyseur, il est recommandé de contrôler l'alimentation en monomère dans le réacteur. Une vitesse moyenne d'alimentation favorable est inférieure ou égale à 500 $Nl.\times h.^{-1}\times g^{-1}$ de catalyseur.

Comme précédemment, le sphéroprotecteur peut être introduit à un stade quelconque du procédé de prépolymérisation, ou encore additionné au prépolymère stocké sous gaz inerte après la prépolymérisation. Lorsque le sphéroprotecteur est ajouté au prépolymère après prépolymérisation, l'opération se fait, sous atmosphère inerte, soit par mélange au prépolymère en suspension dans un liquide inerte soit par imprégnation de la poudre de prépolymère.

Ce sphéroprotecteur est associé dans une proportion préférentielle de 500 à 4000 ppm calculée en aluminium pour 1000 à 25000 ppm de catalyseur dans le prépolymère de degré de prépolymérisation adapté au procédé de polymérisation ultérieur. Le rapport admis en poids de l'aluminium sur le catalyseur est compris entre $30 \cdot 10^{-3}$ et 4.

Dans la mesure où un limitateur de chaîne est utilisé dans la prépolymérisation, on choisit de préférence l'hydrogène.

Le prépolymère obtenu selon l'invention est généralement stocké sous forme sèche en vue d'une utilisation ultérieure comme catalyseur de polymérisation en suspension ou en phase gazeuse pour le fabrication de polyéthylène linéaire en poudre à morphologie sphérique. Ce catalyseur, sous forme de prépolymère à morphologie sphérique, conserve sa morphologie en cours de polymérisation et permet, grâce à cette propriété, d'obtenir des polyéthylènes linéaires possédant également une morphologie sphérique.

Le catalyseur traité selon l'invention est utilisé comme un catalyseur classique dans les procédés de polymérisation en suspension ou en phase gazeuse des oléfines. Bien qu'il puisse être utilisé tel quel il n'est pas exclu, afin de régler sa productivité, d'ajouter en complément un cocatalyseur au milieu réactionnel. Dans ce cas le cocatalyseur peut être un sphéroprotecteur et mieux le sphéroprotecteur mis en oeuvre dans la préparation du prépolymère.

Dans un procédé de polymérisation d'éthylène en suspension, on opère de façon habituelle dans un milieu liquide hydrocarboné à des températures pouvant aller jusqu'à 120°C et sous des pressions pouvant aller jusqu'à 250 bars.

La polymèrisation d'éthylène en phase gazeuse, en présence d'hydrogène et de gaz inerte peut être effectuée dans tout réacteur permettant une polymérisation en phase gazeuse et en particulier dans un réacteur à lit agité ou à lit fluidisé. Les conditions de mise en oeuvre sont connues de l'art antérieur. On opère généralement à une température inférieure au point de fusion Tf du polymère ou copolymère à synthétiser et plus particulièrement entre 20°C et (Tf −5°C) et sous une pression telle que l'éthylène et éventuellement les autres monomères hydrocarbonés présents dans le réacteur soient essentiellement en phase vapeur.

Les exemples suivants illustrent l'invention sans la limiter.

Préalablement, le catalyseur sphérique traité selon l'invention a été préparé dans les conditions ci-après.

Dans un réacteur en verre de 1,5 litre muni d'un moyen d'agitation, on introduit sous azote après séchage et purge 200 mM de n butyl-butyl sec. magnésium et 33 mM de triéthylaluminium, respectivement en solution molaire dans l'heptane. Le contact est maintenu pendant 1 heure à 80°C. On ajoute ensuite sous agitation 200 mM d'éther diisoamylique à 50°C, puis en 2 heures, toujours à 50°C, 550 mM de chlorure de tertiobutyle. On filtre et on lave le solide obtenu, formant support, deux fois avec 400 ml (cc) d'heptane à 50°C.

On remet en suspension le solide dans 400 ml (cc) d'heptane à 80°C et on ajoute en 1 heure 600 mM de $TiCl_4$. On filtre et on lave avec deux fois 400 cc. d'heptane le catalyseur obtenu. Le catalyseur est séché à 70°C sous azote et stocké sous atmosphère inerte. Ce catalyseur solide se présente sous la forme de particules, parfaitement sphériques, de dimension moyenne de 30 μm (microns) environ.

Exemple 1

Préparation du prépolymère

Dans un réacteur de 8,2 l degazé et sec on introduit sous azote et sous agitation de 400 tours/minute successivement à 40°C.

3 l d'hexane sec
6 mM de trihexylaluminium pur (THA)
3 g de catalyseur sphérique précédent en suspension dans 75 ml d'hexane
0,8 bar absolu d'hydrogène
4 bars absolus d'azote, puis:

de l'éthylène à un débit de 30 Nl/h durant 1 h, suivi d'un débit de 60 Nl/h durant 1 h, d'un débit de 130 Nl/h pendant 2 h et enfin de 200 Nl/h pendant 50 minutes.

On arrête l'introduction d'éthylène, la pression totale chute de 6 bars absolus à 5 bars absolus en 5 minutes. L'agitation est arrêtée pour laisser décanter et évacuer le liquide surnageant. Puis après élimination du solvant restant à 50°C sous azote, on extrait la poudre de prépolymère qui est stockée sous atmosphère inerte.

On recueille 650 g de poudre de prépolymère sec à morphologie sphérique, sans agglomèrats, de degré de prépolymérisation de 218 g de prépolymère par gramme de catalyseur de diamètre moyen des particules (dp 50):280 µm (microns).

Préparation des sphéroprotecteurs et du catalyseur sous forme prépolymère actif

Dans un tube de Schlenk on introduit et mélange à l'abri de la lumière, sous agitation et balayage d'azote de l'alkylaluminium pur et un alkylaluminium monochloré dans un rapport molaire donné pour chaque essai sous le tableau I suivant. Les complexes ainsi préparés sont déposés au goutte à goutte sur le prépolymère pulvérulent précédent agité et sous atmosphère inerte. La quantité déposée est telle que la concentration en aluminium sur le prépolymère soit de 2000 ppm.

Copolymérisation du butène-1 et de l'éthylène

On opère dans un réacteur de 8,2 l préalablement séché, en présence de 100 g de poudre de polyéthylène, muni d'un agitateur tournant à une vitesse de 400 tours/minute et maintenu à 85°C pendant toute la polymérisation.

Dans le réacteur maintenu sous vide d'environ 1,33 Pa, on injecte du butène-1 jusqu'à l'obtention d'une pression de 1 bar, puis comme cocatalyseur un certain volume, donné pour chaque essai dans le tableau I suivant, du sphéroprotecteur identique à celui utilisé dans la préparation du prépolymère selon l'invention. On complète l'injection de butène-1 jusqu'à obtenir une pression de 2,5 bars. On injecte ensuite successivement dans le réacteur 1,5 bar d'hydrogène et 13 bars d'éthylène jusqu'à obtenir des pressions partielles d'hydrogène et d'éthylène respectivement égales à 1,5 et 13 bars.

On introduit alors dans le réacteur×g (donnés dans le tableau I) de prépolymère actif préparé précédemment, en effectuant cette introduction par poussée à l'azote avec poursuite de l'injection d'azote jusqu'à ce que la pression totale à l'intérieur du réacteur atteigne 21 bars. On maintient la pression dans le réacteur à cette valeur par injection d'éthylène et de butène-1 dans un rapport molaire butène-1/éthylène égal à 0,0466.

Après 4 h de réaction, la polymérisation est arrêtée par décompression du réacteur. On purge à l'azote et laisse refroidir.

Les conditions opératoires manquantes et les résultats de contrôles effectués sur le polyéthylène linéaire obtenu sont donnés dans le tableau I suivant, sachant que les essais 1, 2, 7, 8, 9 et 10 sont donnés à titre comparatif.

TABLEAU I

| Essai | Poids de prépolymère actif en g | Poids de cataly-seur cor-respon-dant en g | Nature du sphéroprotec-teur | Rapport molaire alkyl Al monochloré /alkyl Al non halo-géné | Cocatalyseur en cc. | densité | Morpho-logie | % des parti-cules de polyé-thylène <200 µm(µ) | Coulabilité sec. | Produc-tion en 4 h | Productivité en g.PE/g.cata |
|---|---|---|---|---|---|---|---|---|---|---|---|
| 1 | 7 | 0,032 | TEA | — | 0,1 | 0,922 | G | 7 | 32 | 958 | 29 940 |
| 2 | 7 | 0,032 | THA | — | 0,2 | 0,920 | G | 1,8 | 32 | 703 | 21 970 |
| 3 | 7 | 0,032 | TEA/DEAC | 8,5 | 0,15 | 0,919 | S | 0,5 | 28 | 594 | 18 560 |
| 4 | 8,3 | 0,038 | THA/DEAC | 8,5 | 0,1 | 0,921 | S | 0,6 | 27 | 1 464 | 38 526 |
| 5 | 8,3 | 0,038 | DEAC | — | 0,1 | 0,919 | S | 0 | 30 | 511 | 13 447 |
| 6 | 7 | 0,032 | THA/DHAC | 8,5 | 0,2 | 0,920 | S | 0,6 | 30 | 351 | 10 970 |
| 7 | 7 | 0,032 | THA/EADC | 0,33 | 0,15 | 0,920 | S/G | 1 | 35 | 197 | 6 160 |
| 8 | 7 | 0,032 | TEA/EADC | 0,16 | 0,15 | 0,921 | S/G | 2 | 33 | 394 | 12 310 |
| 9 | 7 | 0,032 | THA/EASC | 0,055 | 0,15 | 0,920 | G | 5 | 33 | 1 036 | 32 375 |
| 10 | 7 | 0,032 | THA/EADC | 4,25 | 0,15 | | Pas de réaction | | | 0 | 0 |

TEA=triéthylaluminium
THA=trihexylaluminium
DEAC=Diéthylaluminium monochloré
DHAC=Dihexylaluminium monochloré
EADC=éthylaluminium dichloré
EASC=éthylaluminium sesquichloré
G=Granulaire
S=Sphérique

La morphologie des prépolymères des essais 1, 4 et 8 est illustrée par les photos annexées.

Exemple 2

Dans un réacteur de 8,2 l dégazé et sec on introduit sous azote et sous agitation de 330 tr/min successivement à 40°C

3 l d'hexane sec
10 mM de THA
30 mM de DEAC
10 ml (cc) d'une suspension à 88 g/l du catalyseur sphérique précédent soit 0,88 g de catalyseur
0,5 bar d'hydrogène.

On introduit alors pendant 15 mm un mélange d'éthylène et de butène contenant 1,6% molaire de butène à raison de 30 Nl/h. puis pendant 3h15 ce même mélange à raison de 40 Nl/h.

On arrête l'introduction des monomères. L'agitation est arrêtée pour laisser décanter et évacuer le liquide surnageant. Puis après élimination du solvant restant à 50°C sous azote, on extrait la poudre de prépolymère qui est stockée sous atmosphère inerte.

On recueille 235 g de poudre de prépolymère sec à morphologie sphérique, sans agglomérats, de degré de prépolymérisation de 267 g de prépolymère par gramme de catalyseur de diamètre moyen des particules (dp 50):283 µm (microns). Le prépolymère possède une teneur en aluminium de l'ordre de 1000 ppm.

Dans les conditions de l'exemple 1, on polymérise du butène-1 et de l'éthylène en présence du prépolymère actif obtenu.

Les conditions opératoires manquantes et les résultats des contrôles effectués sur le polyéthylène linéaire obtenu sont donnés dans le tableau II suivant.

TABLEAU II

| | |
|---|---|
| Poids de prépolymère actif en g | 10 |
| Poids de catalyseur en g | 0,037 |
| Nature du sphéroprotecteur | THA/DEAC |
| Rapport molaire alkyl Al monochloré/alkyl Al du sphéroprotecteur rajouté dans le réacteur | 8,5 |
| Volume THA/DEAC rajouté dans de réacteur | 0,15 ml (cc) pur |
| Rapport molaire THA/DEAC sur prépolymère | 3 |
| Densité du polyéthylène obtenu | 0,919 |
| % des particules <200 µm (µ) | 0,2 |
| Morphologie | Sphérique |
| Coulabilité en secondes | 28 |
| Production en g en 4 heures | 740 |
| Productivité en g.polyéthylène/g.catalyseur | 20 000 |

Exemple 3

Préparation du prépolymère actif

Dans un réacteur de 8,2 1 muni d'un système d'agitation, on introduit à 50°C sous azote:

3 l d'hexane séché et dégazé à l'azote
29 mM de tri-n-hexylaluminium
40 mM de chlorure de diéthylaluminium
3 bars d'azote

On injecte dans le réacteur 46 g de catalyseur sous forme d'une suspension dans 0,5 l d'hexane. Puis on ajoute respectivement:

0,5 bar d'hydrogène
100 Nl/h d'éthylène durant 1 heure
200 Nl/h d'éthylène durant 1 heure

Le produit formé est séché par strippage à l'azote et l'on recueille sous azote 387 g de catalyseur consolidé de degré d'avancement de 8,4 g PE/g catalyseur.

On mélange intimement sous atmosphère d'azote en boîte à gants, 20 g du catalyseur consolidé, 100 g d'une poudre de polyéthylène et un mélange de 3 millimoles de DEAC et 9 millimoles de THA.

On introduit ce mélange sous azote dans un réacteur de polymérisation de 8,2 l préalabalement séché et dégazé en muni d'un système d'agitation.

On ajoute successivement à 50°C:

4 bars d'azote
0,8 bar d'hydrogène
50 Nl/h d'éthylène durant 30 minutes
100 Nl/h d'éthylène durant 3 heures

On recueille 483 g de poudre dont 383 g de prépolymère ayant pour degré de polymérisation 158 g PE/g catalyseur.

Copolymérisation du butène-1 et de l'éthylène

On engage le prépolymère obtenu dans les conditions de polymérisation de l'exemple 1.

Les conditions opératoires et les résultats des contrôles effectués sur le polyéthylène linéaire obtenu sont donnés dans le tableau III suivant.

TABLEAU III

| | |
|---|---|
| Poids de prépolymère actif en g | 5 |
| Poids de catalyseur en g | 0,031 |
| Nature du sphéroprotecteur | THA/DEAC |
| Rapport molaire alkyl Al monochloré/alkyl Al du sphéroprotecteur rajouté dans le réacteur | 8,5 |
| Volume THA/DEAC rajouté dans le réacteur | 0,3 ml (cc) pur |
| Rapport molaire THA/DEAC sur prépolymère | 2 |
| Densité du polyéthylène obtenu | 0,920 |
| % des particules <200 µm (µ) | 0,6 |
| Morphologie | Sphérique |
| Coulabilité en secondes | 30 |
| Production en g en 4 heures | 1162 |
| Productivité en g.polyéthylène/g.catalyseur | 37480 |

**Revendications**

1. Procédé de traitement de catalyseur sphérique de polymérisation des oléfines contenant au moins un métal de transition, un composé de magnésium et un halogène caractérisé en ce que, en présence du catalyseur sphérique, on prépolymérise en présence d'un co-catalyseur choisi parmi les alkylaluminiums, au moins partiellement en suspension, de l'éthylène jusqu'à un degré de prépolymérisation inférieure à 100 lorsque le prépolymère doit être utilisé ultérieurement comme catalyseur dans un procédé de polymérisation en suspension, ou, jusqu'à un degré de pré-polymérisation supérieure à 50 lorsque le prépolymère doit être utilisé ultérieurement comme catalyseur dans un procédé de polymérisation en phase gazeuse, sans, dans ce dernier ces, que le prépolymère formé représente plus de 10% du polymère final, un sphéroprotecteur étant introduit dans le milieu réactionnel de prépolymérisation ou additionné au

prépolymère après la prépolymérisation, la formule admise dudit sphéroprotecteur étant:

$$Al\ R'_m R''_n Cl_p H_q$$

dans laquelle

$0{,}05 < p \leq 1$
$0 < m < 2{,}95$
$0 < n < 2{,}95$
$0 \leq q \leq 1$

sachant que $m+n+p+q=3$ et R' et R'', identiques ou différents, sont des radicaux hydrocarbonés linéaires, ramifiés ou cycliques contenant de 1 à 14 atomes de carbone.

2. Procédé selon la revendication 1 caractérisé en ce que le sphéroprotecteur résulte du mélange d'au moins un alkylaluminium non halogéné avec au moins un alkylaluminium mono halogéné.

3. Procédé selon l'une des revendications 1 à 2, caractérisé en ce qu'on prépolymérise en suspension jusqu'à un degré d'avancement réduit de polymérisation et que l'on reprend le prépolymère formé dans un système de prépolymérisation en phase gazeuse au degré de prépolymérisation adapté au procédé de polymérisation en suspension ou en phase gazeuse dans lequel le prépolymère doit être ultérieurement mis en oeuvre comme catalyseur.

4. Procédé selon la revendication 3 caractérisé en ce que le degré d'avancement est inférieur à 10 g de polymère par gramme de catalyseur.

5. Procédé selon l'une des revendications 1 à 4 caractérisé en ce que dans la partie de prépolymérisation en phase gazeuse, le monomère est introduit dans le réacteur à un débit moyen, égal ou supérieur à 500 $Nl.\times h^{-1} \times g^{-1}$ de catalyseur sphérique.

6. Procédé selon l'une des revendications 1 à 4, caractérisé en ce que dans la prépolymérisation en suspension, le monomère est introduit dans le réacteur à un débit moyen, égal ou inférieur à 500 $Nl.\times h^{-1} \times g^{-1}$ de catalyseur sphérique.

7. Procédé selon l'une des revendications 1 à 6 caractérisé en ce que le sphéroprotecteur est associé dans une proportion de 500 à 4000 ppm calculée en aluminium pour 1000 à 25000 ppm de catalyseur dans le prépolymère de degré de prépolymérisation adapté au procédé de polymérisation dans lequel le prépolymère sera mis ultériurement en oeuvre.

8. Procédé selon l'une des revendications 1 à 7 caractérisé en ce que le rapport en poids de l'aluminium sur le catalyseur est compris entre $30.10^{-1}$ et 4.

9. Procédé de fabrication en suspension ou en phase gazeuse de polyéthylène linéaire en poudre à morphologie sphérique caractérisé en ce qu'on utilise un catalyseur préparé selon l'une des revendications 1 à 8.

10. Procédé selon la revendication 9 caractérisé en ce qu'on ajoute au milieu réactionnel un cocatalyseur choisi parmi les sphéroprotecteurs.

**Patentansprüche**

1. Verfahren zur Behandlung eines kugelförmigen Katalysators zur Olefinpolymerisation, der mindestens ein Übergangsmetall, eine Magnesiumverbindung und ein Halogen enthält, dadurch gekennzeichnet, daß man in Gegenwart des kugelförmigen Katalysators und in Gegenwart eines aus den Alkylaluminiumverbindungen ausgewählten Cokatalysators das Ethylen wenigstens teilweise in Suspension bis zu einem Vorpolymerisationsgrad kleiner als 100, wenn das Vorpolymer anschließend als Katalysator in einem Suspensionspolymerisations-Verfahren eingesetzt werden soll oder bis zu einem Vorpolymerisationsgrad größer als 50, wenn das Vorpolymere anschließend als Katalysator in einem Gasphasen-Polymerisationsverfahren eingesetzt werden soll, vorpolymerisiert, ohne daß im letzteren Fall das gebildete Vorpolymere mehr als 10% des Endpolymeren ausmacht, und ein Kugelformprotektor in das Reaktionsmilieu der Vorpolymerisation eingeführt oder nach der Vorpolymerisation zum Vorpolymeren hinzugegeben wird und der Kugelformprotektor die hypothetische Formel:

$$Al\ R'_m R''_n Cl_p H_q$$

hat, in der

$0{,}05 < p \leq 1$
$0 < m < 2{,}95$
$0 < n < 2{,}95$
$0 \leq q \leq 1$

und bekannt ist, daß $m+n+p+q=3$ und R' und R'' identische oder verschiedene, geradkettige, verzweigte oder cyclische, 1 bis 14 Kohlenstoffatome enthaltende Kohlenwasserstoffreste sind.

2. Verfahren nach Anspruch 1, dadurch gekennzeichnet, daß der Kugelformprotektor aus einem Gemisch aus mindestens einem nichthalogenierte Alkylaluminium mit mindestens einem monohalogenierten Alkylaluminium besteht.

3. Verfahren nach Anspruch 1 oder 2, dadurch gekennzeichnet, daß man in Suspension bis zu einem weniger fortgeschrittenen Polymerisationsgrad vorpolymerisiert und daß man das gebildete Vorpolymere in einem Gasphasen-Vorpolymerisationssystem derart wieder einsetzt, daß man den Vorpolymerisationsgrad erreicht, der an das Polymerisationsverfahren in Suspension oder in der Gasphase, in das das Vorpolymere nachfolgend als Katalysator eingesetzt werden soll, angepaßt ist.

4. Verfahren nach Anspruch 3, dadurch gekennzeichnet, daß der Polymerisationsgrad kleiner als 10 g Polymeres pro Gramm Katalysator ist.

5. Verfahren nach einem der Ansprüche 1 bis 4, dadurch gekennzeichnet, daß in der Vorpolymerisationsstufe in der Gasphase das Monomere in den Reaktor bei einem mittleren Durchsatz gleich oder größer als 500 $Nl.\times h^{-1}\times g^{-1}$ des kugelförmigen Katalysators zugegeben wird.

6. Verfahren nach einem der Ansprüche 1 bis 4, dadurch gekennzeichnet, daß bei der Suspensionsvorpolymerisation das Monomere in den Reaktor bei einem mittleren Durchsatz gleich oder kleiner als 500 $Nl.\times h^{-1}\times g^{-1}$ des kugelförmigen Katalysators zugegeben wird.

7. Verfahren nach einem der Ansprüche 1 bis 6, dadurch gekennzeichnet, daß der Kugelformprotektor in einem Anteil von 500 bis 4000 ppm, berechnet als Aluminium, auf 1000 bis 25000 ppm des Katalysators an das Vorpolymere mit einem an das Polymerisationsverfahren, in dem es nachfolgend eingesetzt werden soll, angepaßten Vorpolymerisationsgrad, gebunden ist.

8. Verfahren nach einem der Ansprüche 1 bis 7, dadurch gekennzeichnet, daß das Gewichtsverhältnis des Aluminiums zum Katalysator zwischen $30 \cdot 10^{-3}$ und 4 beträgt.

9. Verfahren zur Herstellung von geradkettigem Polyethylen als kugelförmiges Pulver in Suspension oder in der Gasphase, dadurch gekennzeichnet, daß man einen nach einem der Ansprüche 1 bis 8 hergestellten Katalysator einsetzt.

10. Verfahren nach Anspruch 9, dadurch gekennzeichnet, daß man zum Reaktionsmilieu einen aus den Kugelformprotektoren ausgewählten Cokatalysator hinzugibt.

**Claims**

1. A process for the treatment of a spherical catalyst for the polymerization of olefins, containing at least one transition metal, a magnesium compound and a halogen, characterized in that ethylene is prepolymerized in the presence of a cocatalyst selected from the alkyl aluminiums and at least partially in suspension, to a degree of prepolymerization lower than 100 where the prepolymer is to be subsequently used as a catalyst in a process of polymerization in suspension, or to a degree of prepolymerization higher than 50 when the prepolymer is to be subsequently used as a catalyst in a process of polymerization in the gaseous phase without, in the latter case, the prepolymer formed representing more than 10% of the final polymer, a sphere-protector being introduced into the prepolymerization reaction medium or added to the prepolymer after prepolymerization, the formula adopted for said sphere-protector being:

$$Al\ R'_mR''_nCl_pH_q$$

where

$0.05<p\leq1$
$0<m<2.95$
$0<n<2.95$
$0\leq q\leq1$

knowing that $m+n+p+q=3$ and R' and R'', which are identical or different, are linear, branched or cyclic hydrocarbon radicals containing from 1 to 14 carbon atoms.

2. A process according to Claim 1, characterized in that the sphere-protector results from the mixing of at least one halogenated alkyl aluminium with at least one monohalogenated alkyl aluminium.

3. A process according to one of Claims 1 and 2, characterized in that prepolymerization is performed in suspension to a reduced degree of advance of polymerization and the prepolymer formed is taken up in a system for prepolymerization in the gaseous phase to the degree of prepolymerization suitable for the polymerization process in suspension or in the gaseous phase in which the prepolymer will subsequently be used as a catalyst.

4. A process according to Claim 3, characterized in that the degree of advance is less than 10 g of polymer per gram of catalyst.

5. A process according to one of Claims 1 to 4, characterized in that in the prepolymerization stage in the gaseous phase the monomer is introduced into the reactor at an average rate equal to or lower than 500 $Nl.\times h^{-1}\times g^{-1}$ of spherical catalyst.

6. A process according to one of Claims 1 to 4, characterized in that in the prepolymerization in suspension the monomer is introduced into the reactor at an average rate equal to or lower than 500 $Nl.\times h^{-1}\times g^{-1}$ of spherical catalyst.

7. A process according to one of Claims 1 to 6, characterized in that the sphere-protector is associated in a proportion of 500 to 4000 ppm calculated in aluminium to 1000 to 25 000 ppm of catalyst in the prepolymer of degree of prepolymerization suitable for the polymerization process in which the prepolymer will be subsequently used.

8. A process according to one of Claims 1 to 7, characterized in that the aluminium:catalyst weight ratio is between $30.10^{-3}$ and 4.

9. A process for the production in suspension or in the gaseous phase of pulverulent linear polyethylene of spherical morphology, characterized in that a catalyst is used which is prepared according to one of Claims 1 to 8.

10. A process according to Claim 9, characterized in that a cocatalyst selected from the sphere-protectors is added to the reaction medium.

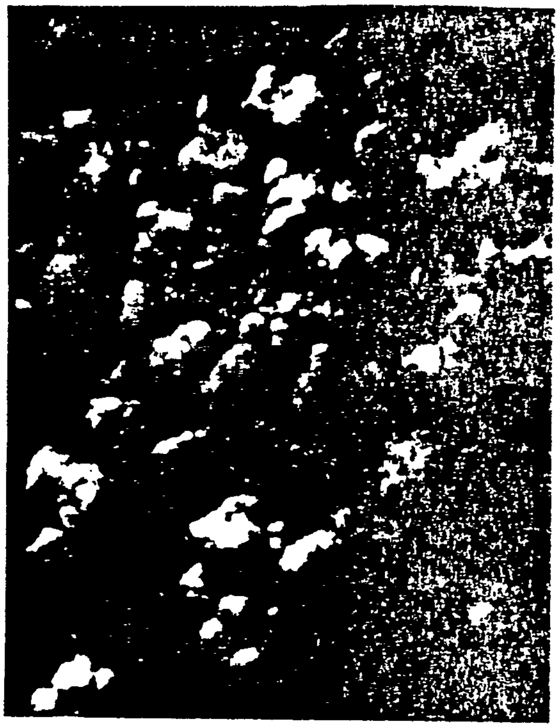

ESSAI 1

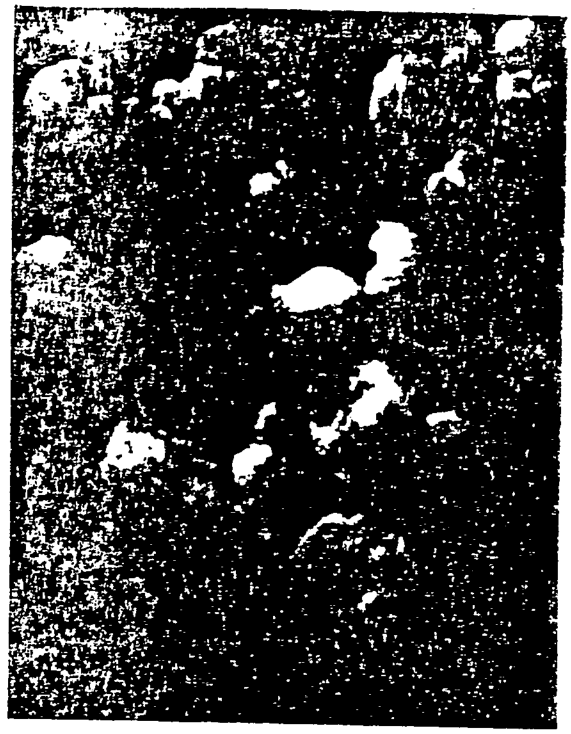

ESSAI 8

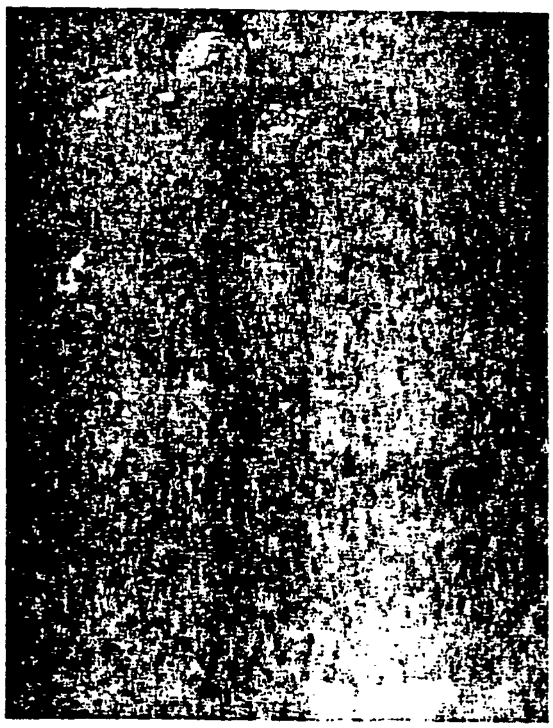

ESSAI 4